# EUROPÄISCHE PATENTSCHRIFT

(11) **EP 0 908 743 B1**
(45) Veröffentlichungstag und Bekanntmachung des Hinweises auf die Patenterteilung: **05.07.2006**
(21) Anmeldenummer: 98117785.0
(22) Anmeldetag: 18.09.1998
(51) Int. Cl.: G01T 1/24

(54) **Röntgendetektor**
X-RAY detector
Détecteur de rayonnement X

(30) Priorität: 01.10.1997 DE 19743525
(43) Veröffentlichungstag der Anmeldung: 14.04.1999
(73) Patentinhaber: SIEMENS AKTIENGESELLSCHAFT, 80333 München (DE)
(72) Erfinder: Sklebitz, Hartmut,Dipl.-Ing.(FH), 91056 Erlangen (DE); Hoheisel, Martin, Dr. Dipl.-Phys., 91056 Erlangen (DE)

(56) Entgegenhaltungen:
- US-A- 4 709 382
- US-A- 4 905 269
- US-A- 4 914 301
- US-A- 5 555 284
- PATENT ABSTRACTS OF JAPAN vol. 010, no. 149 (P-461), 30. Mai 1986 (1986-05-30) -& JP 61 003082 A (HITACHI MEDEIKO:KK), 9. Januar 1986 (1986-01-09)

## Beschreibung

Die Erfindung betrifft einen Röntgendetektor zur Erfassung eines Röntgenstrahlenbildes. Derartige Röntgendetektoren bestehen beispielsweise aus einem Szintillator und einer Anordnung zur Erfassung des erzeugten optischen Bildes. Derartige Röntgendetektoren werden in Röntgendiagnostikeinrichtungen für medizinische Zwecke eingesetzt.

Die heute auf dem Markt erhältlichen Röntgendetektoren haben meist eine quadratische oder rechteckige Form. So weisen Röntgenfilme mit Verstärkerfolie und Speicherfoliensysteme ein rechteckiges Format auf. Die in der EP 0 189 710 beschriebenen, in Entwicklung befindlichen Festkörper-Matrixdetektoren aus Caesiumjodid (CsI) und amorphem Silizium (a-Si) oder, wie in der US 5,313,066 beschrieben, aus Selen (Se) und a-Si haben ein rechteckiges quadratisches Format. Röntgen-Bildverstärker haben dagegen eine runde Eintrittskalotte, so daß sich ein kreisförmiges Röntgenbild ergibt.

Es gibt Röntgenaufnahmen, bei denen es wünschenswert ist, mit dem Röntgen-Aufnahmesystem möglichst nahe an den menschlichen Körper heranzukommen. So möchte man beispielsweise bei mammographischen Aufnahmen das Gewebe nahe der Brustwand untersuchen. Bei Thorax-Aufnahmen sollte auch der Hals der Patienten abgebildet werden können. Zu derartigen Zwecken sind die vorhandenen Röntgen-Aufnahmesysteme mit ihren Formaten nicht optimal geeignet.

Bisher wurden die Röntgen-Aufnahmesysteme so konstruiert, daß die für Röntgenstrahlen empfindliche Fläche mindestens an einer Seite bis an den Rand des Detektors reicht. So beträgt der Abstand vom Röntgenfilm in der Film-Folien-Kassette einer mammographischen Diagnostikeinrichtung zur Brustwand nur wenige Millimeter. Es ist aber nur an einer Stelle möglich, so nahe an die Brustwand der Patientin heranzukommen.

Aus der US 4,905,269 ist eine Röntgenfilmbussette bekannt, die mit Ausschnitten versehen ist, so dass sie sich einer vorgegebenen Kontur anpasst.

Die Erfindung geht von der Aufgabe aus, einen Röntgendetektor der eingangs genannten Art zu schaffen, bei dem die Matrix der Bildgebung dienenden Bereiche möglichst nahe an den Körper einer Untersuchungsperson ragen, so daß auch Untersuchungen von schwer erreichbaren Stellen möglich sind.

Die Aufgabe wird durch die Ausbildung des Röntgendetektors gemäß dem kennzeichenden Merkmalen des PA 1 gelöst. Dadurch kann der Röntgendetektor möglichst gut an die anatomischen Gegebenheiten angepaßt werden, so daß er über weite Bereiche unmittelbar bis an den Körper eines Patienten reicht.

Es hat sich als vorteilhaft erwiesen, wenn der Röntgendetektor derart ausgebildet ist, daß sich die Kontur der gebogenen Seite der Anatomie des zu untersuchenden Körperteiles eines durchschnittlichen Patienten anpaßt.

Erfindungsgemäß kann der Röntgendetektor einen konkaven Ausschnitt aufweisen. Der Röntgendetektor kann an unterschiedlich geformte Körper oder -teile angepaßt werden, wenn er asymmetrisch ausgebildet ist. Dabei kann der Röntgendetektor derart ausgebildet sein, daß zwei benachbarte Seiten, die rechtwinklig aufeinanderstehen, konkave Ausschnitte aufweisen, die mit unterschiedlichen Konturen versehen sein können.

Es hat sich als vorteilhaft erwiesen, wenn der Röntgendetektor derart ausgebildet ist, daß wenigstens eine der Seiten des Röntgendetektors einen konvexen Ausschnitt aufweist.

Erfindungsgemäß kann der Röntgendetektor derart ausgebildet sein, daß eine seiner Seiten einen Hals-Ausschnitt aufweist.

Die Erfindung ist nachfolgend anhand von in der Zeichnung dargestellten Ausführungsbeispielen näher erläutert. Es zeigen:
- Figur 1: eine erfindungsgemäße Matrix mit einem konkav gebogenen Ausschnitt,
- Figur 2: eine erfindungsgemäße Matrix mit einem asymmetrischen Ausschnitt und
- Figur 3: mit zwei asymmetrischen konkaven Ausschnitten,
- Figur 4: eine erfindungsgemäße Matrix mit einem konkav und einem konvex gebogenen Ausschnitt und
- Figur 5: eine erfindungsgemäße Matrix mit einem halbrundem Hals-Ausschnitt.

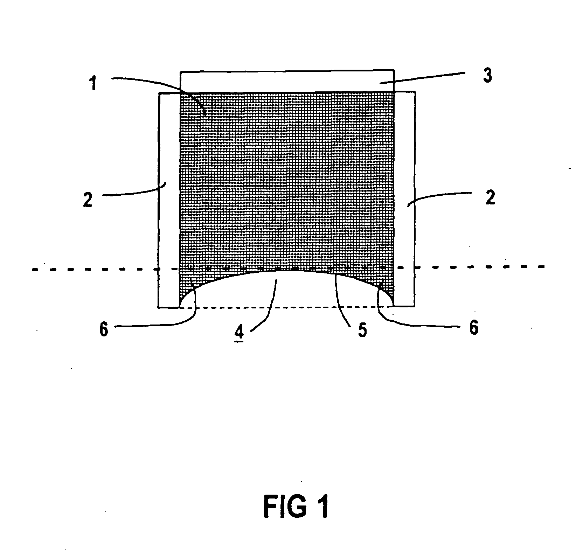

Eine erste Ausführung der erfindungsgemäßen Matrix 1 ist in der Figur 1 dargestellt. Auf einem rechteckigen Glassubstrat werden Photodioden, Schaltelemente, beispielsweise Dünnfilmtransistoren oder Schaltdioden, und Leiterbahnen durch die bekannten Dünnfilm-Depositionstechniken und photolithographische Strukturierung derart aufgebracht, daß sie ein matrixförmiges Array von n Reihen und m Spalten von Bildelementen bilden. Dabei werden die Photodioden in vorteilhafter Weise aus amorphem Silizium (a-Si) hergestellt.

Die Leiterbahnen für die Ansteuerung der einzelnen Bildpunkte führen zu zwei Seiten der rechteckförmigen Matrix 1 und sind dort mit den Ansteuerschaltkreisen 2 verbunden, über welche die Schalter der einzelnen Bildpunkte mit Spannungspulsen in bekannter Weise versorgt werden. Die erzeugten Signalladungen werden aus den Bildpunkten über Leiterbahnen ausgelesen, von an einer dritten Seite der Matrix 1 angeordneten Ausleseverstärkerschaltungen 3 verstärkt und der weiteren Verarbeitung zugeführt.

An der vierten Seite der Matrix 1 ist ein erfindungsgemäßer Ausschnitt 4 mit gebogener Kontur vorgesehen, die beispielsweise konkav sein kann. Diese Kontur des gebogenen Ausschnittes 4 kann der Anatomie des zu untersuchenden Körperteiles eines durchschnittlichen Patienten angepaßt werden.

Zur Herstellung des gebogenen Ausschnittes 4 wird entlang einer gewünschten Schnittkante 5 beispielsweise durch Laserperforation mittels eines Excimer-Lasers eine Folge von kleinen Löchern mit einem Laserstrahl in der Glasoberfläche erzeugt. Diese Löcher können durchgehend oder als Sacklöcher ausgeführt sein. Die Löcher können beispielsweise einen Durchmesser von 50 µm und einen Abstand von 100 µm haben und eine Tiefe aufweisen, welche die Hälfte der Dicke des Glassubtrates beträgt. Die Löcher müssen die Leiterbahnen, Photodioden und Schalter vollständig durchtrennen, so daß bei diesem Prozeßschritt keine Kurzschlüsse zwischen benachbarten Leiterbahnen auftreten können. Anschließend wird das Glassubstrat, das entlang der vorbestimmten Schnittkante 5 perforiert worden ist, sehr präzise gebrochen.

Wie der Figur 1 weiter zu entnehmen ist, sind die Ansteuerschaltkreise 2 an zwei Seiten der Matrix 1 angeordnet, so daß sichergestellt ist, daß auch in den Seitenbereichen 6 seitlich der Schnittkante 5 jeder Bildpunkt an einem Ansteuerschaltkreis 2 angeschlossen ist.

Der so erzeugte Sensor wird danach mit Cäsiumjodid bedampft, um ihn gegenüber Röntgenstrahlung empfindlich zu machen. Alternativ kann der Sensor auch schon vor der Formgebung mit einem Szintillator versehen werden und anschließend mit diesem zur Bildung des Ausschnittes 4 zusammen gebrochen werden.

Der Vorteil eines derartigen Herstellungsverfahrens liegt darin, daß erst nach der Herstellung des rechteckigen Sensors entschieden werden kann, welche Form der anatomische Detektor haben soll. Es ist somit denkbar, ausgehend von ein und demselben Sensordesign einen rechteckigen Detektor und mehrere unterschiedlich geformte Detektoren zu erzeugen, so daß sich eine erhebliche Kostenersparnis ergibt.

In einem weiteren Herstellungsverfahren wird die vorgesehene Bruchlinie bereits bei der photolithographischen Strukturierung berücksichtigt. Dabei läßt man die Leiterbahnen kurz vor der späteren Schnittkante 5 des Glassubstrates enden. Durch diese Anordnung ist es möglich, die Sensoren schon vor der Formgebung zu prüfen. Die Gefahr einer Beeinflussung der elektrischen Eigenschaften durch den Laserprozeß ist hier geringer. Allerdings ist man hier auf eine bestimmte Form festgelegt.

In einem dritten Herstellungsverfahren wird der Detektor nicht mit Cäsiumjodid bedampft. Vielmehr werden die einzelnen Bildpunkte aus Schaltern in Dünnfilmtechnik aufgebaut, oberhalb derer eine Halbleiterschicht zur direkten Absorption von Röntgenstrahlung angeordnet ist. In dieser Halbleiterschicht werden von den absorbierten Röntgenquanten elektrische Ladungen erzeugt. Eine solche Schicht kann beispielsweise aus Selen bestehen. Die Strukturierung eines derartigen Detektors erfolgt dann analog zu den oben beschriebenen Herstellungsverfahren.

Auch kann statt Cäsiumjodid als Szintillator Gadoliniumoxisulfid oder ein anderes geeignetes Material Verwendung finden.

Bei anderen Ausführungsformen können beispielsweise die Anschlüsse der Ansteuerschaltkreisen 2 und Ausleseverstärkerschaltungen 3 vertauscht sein.

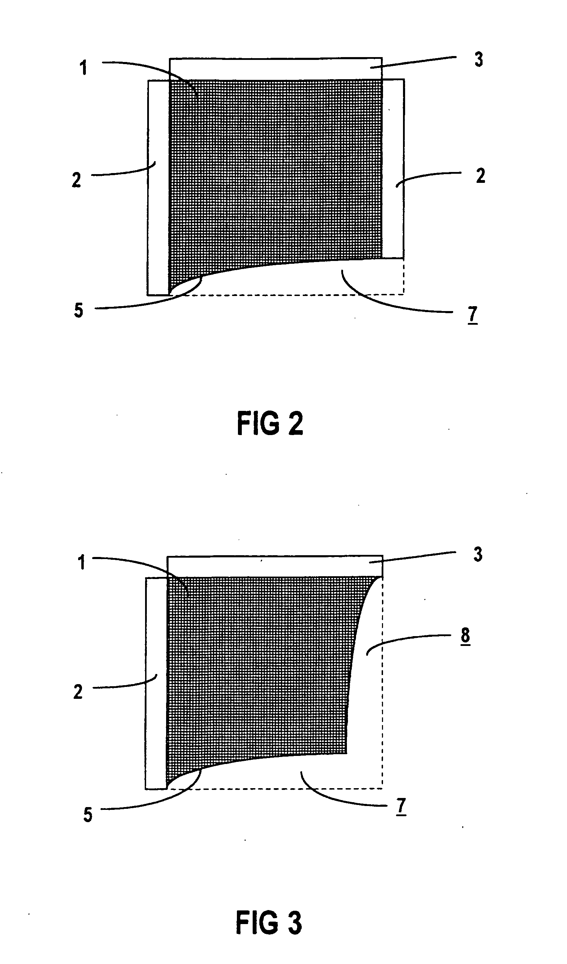

Erfindungsgemäß kann, wie in Figur 2 dargestellt, die Form des Detektors asymmetrisch sein, so daß sich ein asymmetrischer, konkaver Ausschnitt 7 ergeben kann.

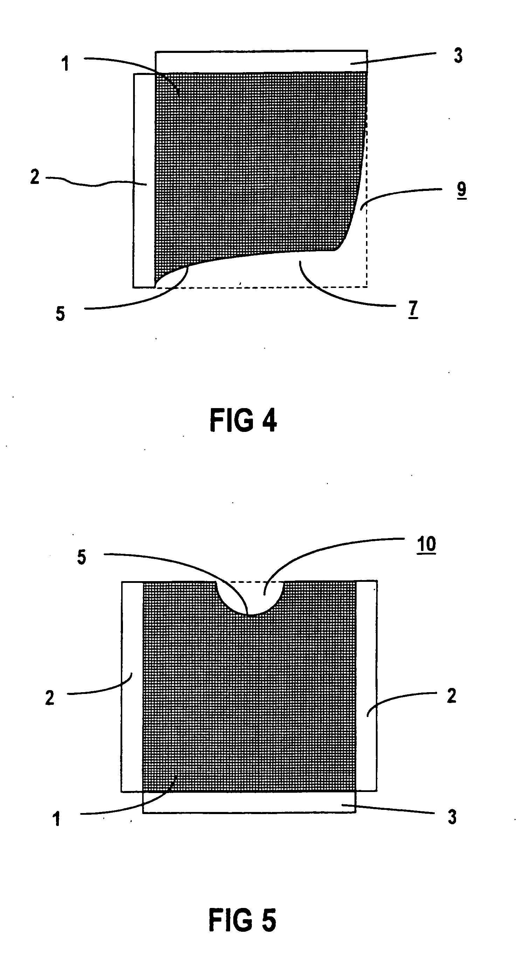

Eine Weiterentwicklung des erfindungsgemäßen Prinzips ist möglich, wenn die Ansteuerleitungen derart geführt sind, daß nur an einer Seite des Detektors Ansteuerschaltkreise 2 notwendig sind. Dann kann die Matrix 1 auch an zwei benachbarten Seiten, konkav geformt sein, wie dies der Figur 3 zu entnehmen ist. Durch die beiden asymmetrisch konkaven Ausschnitte 7 und 8, die unterschiedlich ausgebildet sein können, ist eine Anpassung an die verschiedensten Körperformen auch bei unterschiedlichen Aufnahmerichtungen möglich. Alternativ kann die Matrix 1 auch einen in Figur 4 dargestellten konvexen Ausschnitt 9 aufweisen.

Speziell für Thorax-Aufnahmen ist nur ein Teil der einen Seite der Matrix 1 mit einer derartig in deren Mitte angeordneten Schnittkante 5 versehen, so daß sich ein in Figur 6 dargestellter halbrunder Hals-Ausschnitt 10 ergibt.

Das Format von Röntgendetektoren war traditionell auf die geometrischen Formen "Rechteck" oder "Kreis" beschränkt. Mit dieser erfindungsgemäßen Ausbildung des Röntgendetektors wird diese Einschränkung aufgehoben, so daß neue Röntgenanlagen realisierbar sind, die der menschlichen Anatomie besser anpaßbar sind.

Insbesondere in der Mammographie wird durch einen konkav geformten Röntgendetektor ermöglicht, die Bildgebung in der unmittelbaren Nähe der Brustwand erheblich zu verbessern. Dies ist diagnostisch von erheblichem Nutzen.

Durch die erfindungsgemäße Anordnung der Anschlußleitungen der Matrix 1 nur auf drei Seiten kann an der vierten Seite der gebogene Ausschnitt 4, 7 oder 10 angebracht werden. An zwei Seiten sind die Ansteuerschaltkreise 2 angeordnet, so daß auch die Seitenbereiche angesteuert werden können. Die erzeugten Signalladungen werden an der dritten Seite von Ausleseverstärkerschaltungen 3 verstärkt. An der vierten Seite sind keine Schaltungen, sondern der erfindungsgemäßen konkaven Ausschnitt 4 vorgesehen, wodurch die Form des Ausschnitts entsprechend der jeweiligen Anwendung an den Körper eines durchschnittlichen Patienten angepaßt werden kann.

## Patentansprüche

1. Röntgendetektor zur Erfassung eines Röntgenstrahlenbildes und Umwandlung desselben in eine elektrische Signalfolge, welcher einen Halbleiter-Bildwandler enthält, der in einer rechteckförmigen Matrix (1) angeordnete strahlenempfindliche Zellen sowie über Leitungen mit den Zellen verbundene Ansteuerschaltkreise (2) und Ausleseverstärkerschaltungen (3) aufweist,**dadurch gekennzeichnet, dass** wenigstens eine der Seiten des Röntgendetektors (1) und des Halbleiterbildwandlers einen Ausschnitt (4, 7 bis 10) mit gebogener Kontur aufweist, dass dass an der dem Ausschnitt(4, 7 bis 10) gegenüber liegenden Seite Ansteuerschaltkreise (2) oder Ausleseverstärkerschaltungen (3) als Treiberschaltung und an der oder den übrigen Seiten die jeweils andere Treiberschaltung vorgesehen sind, so dass alle Zellen sowohl an einen Ansteuerschaltkreis (2) als auch an eine Ausleseverstärkerschaltung (3) angeschlossen sind.

2. Röntgendetektor nach Anspruch 1, **dadurch gekennzeichnet, daß** der Röntgendetektor (1) derart ausgebildet ist, daß sich die Kontur der gebogenen Seite der Anatomie des zu untersuchenden Körperteiles eines durchschnittlichen Patienten anpaßt.

3. Röntgendetektor nach Anspruch 1 oder 2, **dadurch gekennzeichnet, daß** der Röntgendetektor (1) einen konkaven Ausschnitt (4) aufweist.

4. Röntgendetektor nach einem der Ansprüche 1 bis 3, **dadurch gekennzeichnet , daß** der Röntgendetektor (1) asymmetrisch ausgebildet ist (Fig. 2).

5. Röntgendetektor nach einem der Ansprüche 1 bis 4, **dadurch gekennzeichnet, daß** der Röntgendetektor (1) derart ausgebildet ist, daß zwei benachbarte Seiten konkave Ausschnitte (7, 8) aufweisen (Fig. 3).

6. Röntgendetektor nach einem der Ansprüche 1 bis 4, **dadurch gekennzeichnet, daß** der Röntgendetektor (1) derart ausgebildet ist, daß wenigstens eine der Seiten des Röntgendetektors (1) einen konvexen Ausschnitt (9) aufweist (Fig. 4).

7. Röntgendetektor nach einem der Ansprüche 1 bis 6 **dadurch gekennzeichnet, daß** der Röntgendetektor (1) derart ausgebildet ist, daß eine der Seiten des Röntgendetektors (1) einen Hals-Ausschnitt (10) aufweist (Fig. 5).

## Claims

1. X-ray detector for recording an X-ray image and converting it into an electrical signal sequence, which contains a semiconductor image converter that has radiation-sensitive cells arranged in a rectangular matrix (1) and control circuits (2) and readout amplifier circuits (3) connected to the cells via lines, **characterized in that** at least one of the sides of the X-ray detector (1) and the semiconductor image converter has a recess (4, 7 to 10) with a curved contour, **in that** control circuits (2) or readout amplifier circuits (3) are provided as one driver circuit on the side opposite the recess (4, 7 to 10) and the respective other driver circuit is provided on the other side or sides, so that all the cells are connected both to a control circuit (2) and to a readout amplifier circuit (3).

2. X-ray detector according to Claim 1, **characterized in that** the X-ray detector (1) is designed so that the contour of the curved side matches the anatomy of an average patient's body part to be examined.

3. X-ray detector according to Claim 1 or 2, **characterized in that** the X-ray detector (1) has a concave recess (4).

4. X-ray detector according to one of Claims 1 to 3, **characterized in that** the X-ray detector 1 is asymmetrically designed (Fig. 2).

5. X-ray detector according to one of Claims 1 to 4, **characterized in that** the X-ray detector (1) is designed so that two neighbouring sides have concave recesses (7, 8) (Fig. 3).

6. X-ray detector according to one of Claims 1 to 4, **characterized in that** the X-ray detector (1) is designed so that at least one of the sides of the X-ray detector (1) has a convex recess (9) (Fig.4).

7. X-ray detector according to one of Claims 1 to 6, **characterized in that** the X-ray detector (1) is designed so that one of the sides of the X-ray detector (1) has a neck recess (10) (Fig.5) .

## Revendications

1. Détecteur de rayons X pour la détection d'une image de rayons X et sa transformation en une suite de signaux électriques, qui comporte un convertisseur d'image à semi-conducteur qui a des cellules sensibles au rayonnement disposées suivant une matrice (1) rectangulaire, ainsi que des circuits (2) de commande reliés aux cellules par des lignes et des circuits (3) amplificateurs de lecture, **caractérisé en ce qu'**au moins l'un des côtés du détecteur (1) de rayons X et du convertisseur d'image à semi-conducteur a une partie (4, 7 à 10) de contour courbée, **en ce qu'**il est prévu sur le côté opposé à la partie (4, 7 à 10) des circuits (2) de commande ou des circuits (3) amplificateurs de lecture comme circuit d'attaque et sur le ou les autres côtés respectivement l'autre circuit d'attaque, de sorte que toutes les cellules sont raccordées tant à un circuit (2) de commande qu'également à un circuit (3) amplificateur de lecture.

2. Détecteur de rayons X suivant la revendication 1, **caractérisé en ce que** le détecteur (1) de rayons X est constitué de façon à ce que le contour du côté courbé s'adapte à l'anatomie de la partie du corps à examiner d'un patient moyen.

3. Détecteur de rayons X suivant la revendication 1 ou 2, **caractérisé en ce que** le détecteur (1) de rayons X a une partie (4) concave.

4. Détecteur de rayons X suivant l'une des revendications 1 à 3, **caractérisé en ce que** le détecteur (1) de rayons X est dissymétrique (Figure 2).

5. Détecteur de rayons X suivant l'une des revendications 1 à 4, **caractérisé en ce que** le détecteur (1) de rayons X est constitué de manière à ce que deux côtés voisins aient des parties (7, 8) concaves (Figure 3) .

6. Détecteur de rayons X suivant l'une des revendications 1 à 4, **caractérisé en ce que** le détecteur (1) de rayons X est constitué de manière à ce qu'au moins l'un des côtés du détecteur (1) de rayons X ait une partie (9) convexe (Figure 4).

7. Détecteur de rayons X suivant l'une des revendications 1 à 6, **caractérisé en ce que** le détecteur (1) de rayons X est constitué de façon à ce que l'un des côtés du détecteur (1) de rayons X ait une partie (10) en forme de col (Figure 5).
